# EUROPÄISCHE PATENTSCHRIFT

(11) **EP 0 729 032 B1**
(45) Veröffentlichungstag und Bekanntmachung des Hinweises auf die Patenterteilung: **15.05.2002**
(21) Anmeldenummer: 96102230.8
(22) Anmeldetag: 15.02.1996
(51) Int. Cl.: G01N 33/92

(54) **Verfahren und Reagenz zur spezifischen Bestimmung von LDL in Serumproben**
Method and reagent for specific determination of LDL in serum samples
Procédé et agent pour la détermination spécifique de LDL en échantillons de sérum

(30) Priorität: 21.02.1995 DE 19505894
(43) Veröffentlichungstag der Anmeldung: 28.08.1996
(73) Patentinhaber: Roche Diagnostics GmbH, 68305 Mannheim (DE)
(72) Erfinder: Karl, Johann, Dr., D-82380 Peissenberg (DE)

(56) Entgegenhaltungen:
- EP-A- 0 076 211
- DE-A- 4 223 355
- US-A- 5 290 703
- BURSTEIN ET AL: "Lipoprotein-polyanion-metal interactions." ADVANCES IN LIPID RESEARCH, Bd. 11, 1973, NEW YORK, NY, Seiten 67-108, XP002056110

## Beschreibung

Die Erfindung betrifft ein Verfahren zur spezifischen Bestimmung von LDL in biologischen Flüssigkeiten in Gegenwart eines LDL fällenden Mittels bzw. eines wasserlöslichen Polymeren, insbesondere eines Polyanions und eines Detergenzes bzw. eines Detergenzgemisches sowie ein hierfür geeignetes Reagenz mit dem Vorteil einer deutlich reduzierten VLDL-Störung. Bei dem Polymeren handelt es sich insbesondere um solche mit verzweigter Struktur, wobei die Seitenäste Säuregruppen, insbesondere verzweigte Alkansulfongruppen, aufweisen.

Die Bestimmung des LDL-Spiegels im Plasma bzw. insbesondere im Serum hat hohe klinische Bedeutung: Bis zu 80 % des Gesamtcholesterins werden in Form der sogenannten Low Density-Lipoproteine (LDL oder β-Fraktion) transportiert und sind damit Bestandteil jener Partikel, die heute als bedeutendste atherogene Komponente im Fettstoffwechsel gelten. Darüber hinaus ist bekannt, daß von erhöhten Plasma-LDL-Konzentrationen eine direkte endothel-schädigende Wirkung zu erwarten ist, die mindestens vergleichbar ist mit einer Reihe anderer Noxen, wie beispielsweise Hypertonie, Hyperinsulinämie oder Endotoxine, und daß das in einem atherosklerotischen Plaque zur Ablagerung gelangte Cholesterin ursprünglich in Form von LDL-Cholesterin im Plasma vorlag.

Eine Reihe Verfahren zur quantitativen Bestimmung von LDL- bzw. β-Cholesterin im Plasma bzw. insbesondere im Serum stehen heute zur Verfügung (Mills, G.L. Lane, P.A., Weech, P.K., A guidebook to lipoprotein technique, Elsevier, Amsterdam, 1984; Cremer, P., und Seidel, D., Deutsch. Gesell. Klin. Chem. Mittl. 21 (1990), 215 - 232).

Man unterscheidet im wesentlichen zwischen differenzierenden Techniken wie Ultrazentrifügation und Elektrophorese auf der einen und Fällungstechniken auf der anderen Seite. Erstere sind dabei mit dem Umstand verbunden, daß sie eine aufwendige Geräteausstattung erfordern und dazu zeitintensiv sind. Sie finden daher heute in erster Linie als Bezugs- bzw. Bestätigungsverfahren Verwendung. Die Fällung mit hochmolekularen, meist negative Ladungen enthaltenden Substanzen zählt dagegen zu den routinetauglichen Laborverfahren. Insbesondere werden heute in der klinischen Diagnostik Dextransulfat, Polyvinylsulfat, polyzyklische, oberflächenaktive Anionen oder Heparin als LDL-Präzipitationsreagenzien eingesetzt. Die Verfahren sind jedoch mit Nachteilen verbunden. So werden bei der Fällung mit Heparin im sauren Medium bzw. mit polyzyklischen Anionen bei Serumproben mit vorausgegangener oder bestehender lipolytischer Aktivität falsch hohe Meßergebnisse für LDL-Cholesterin erhalten. Mit Dextransulfat bzw. Polyvinylsulfat (PVS) - welche heute als Mittel der Wahl gelten, da sie die höchste Präzision bei der LDL-Bestimmung zeigen - werden dagegen in Serumproben mit vorausgegangener oder bestehender lipolytischer Aktivität falsch niedrige Werte gefunden. Außerdem werden mit Dextransulfat-haltigen Präzipitationsreagenzien auch bei hohen Spiegeln freier Fettsäuren bzw. Triglyceriden in der zu untersuchenden Probe falsch niedrige Meßergebnisse erhalten. Diese Nachteile lassen sich auch durch den Zusatz eines entsprechenden Polyanions in Kombination mit einem Detergenz und gegebenenfalls einem zweiwertigen Kation nicht vollständig vermeiden (EP 0 076 211). Die Störung in triglyceridreichen Proben wird primär durch das Vorhandensein hoher VLDL-Konzentrationen verursacht. Dieser Umstand tritt verstärkt bei Heparin-therapierten Personen auf. Weiter ist bei sämtlichen Präzipitationsverfahren nachteilig, daß neben LDL auch das LDL-ähnliche Lipoprotein Lp(a) vollständig mitgefällt wird und die Verfahren somit nicht völlig LDL-spezifisch sind.

In DE 42 23 355 wird ein Verfahren zur spezifischen Bestimmung von LDL mittels bestimmter Alkansulfonsäuregruppen aufweisender Polymere beschrieben. Dieses Verfahren genügt jedoch den heutigen Ansprüchen - insbesondere, wenn die Proben einen hohen Anteil an VLDL aufweisen - nur bedingt.

Der Erfindung lag daher der Aufgabe zugrunde, ein einfach und rasch durchzuführendes Verfahren zur spezifischen Bestimmung von LDL zur Verfügung zu stellen, das weder durch Lipoproteinpartikel, insbesondere VLDL, noch durch erhöhte Konzentrationen von Triglyceriden oder freien Fettsäuren oder durch heparinisierte Proben gestört wird.

Gelöst wird die Aufgabe dadurch, daß der zu untersuchenden Probe ein LDL fällendes Mittel oder ein wasserlösliches Polymeres, beispielsweise mit anionischen Seitenästen sowie ein Detergenz bzw. Detergenzgemisch der allgemeinen Formel (II): 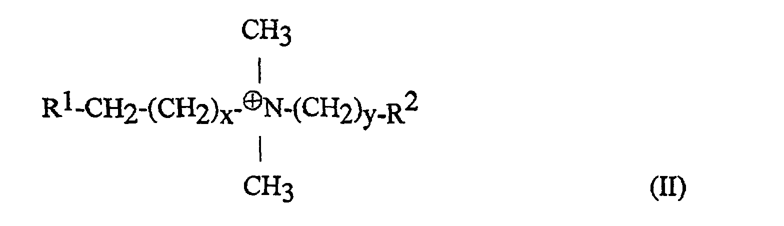 wobei x eine Zahl von 1 bis 20, y eine Zahl von 0 bis 8,
R¹ Wasserstoff, eine Cholamidogruppe oder eine über einen Phosphatrest gebundene Mono- oder Di-Glyceridgruppe ist,
R² Wasserstoff, eine Hydroxygruppe bzw. ein Oxyanion, ein Säurerest, eine Hydroxyalksulfonsäuregruppe oder eine Carboxylgruppe ist,
zugesetzt wird und anschließend der Gehalt des gebildeten LDL-spezifischen Aggregats (Agglutinats) direkt turbidimetrisch bestimmt wird. Auch bei den gängigen Routinemethoden wie beispielsweise Dextran oder PVS als Fällungsmittel kann durch den Zusatz eines erfindungsgemäßen Detergenzies oder Detergenzgemisches die VLDL-Störung vermindert werden.

Als wasserlösliche Polymere können solche mit anionischen Seitengruppen gemäß DE 42 23 355, die sich von Acrylsäureestermonomeren ableiten, aber auch Dextransulfat und Heparin verwendet werden. Bevorzugt haben sich Verbindungen der allgemeinen Formel (I) erwiesen: 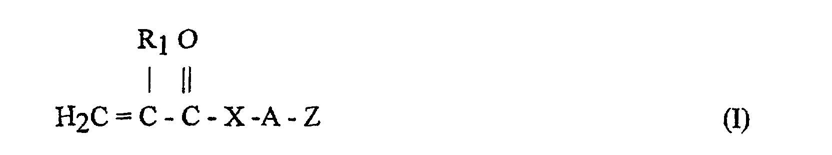 in der die Reste R¹ Wasserstoff oder ein niederer Kohlenwasserstoffrest, vorzugsweise eine Methylgruppe, X ein Sauerstoffatom oder eine NH-Gruppierung, A eine lineare der verzweigte Kohlenwasserstoffkette bestehend aus 2 bis 10 C-Atomen, wobei verzweigte Ketten bevorzugt sind, und Z eine 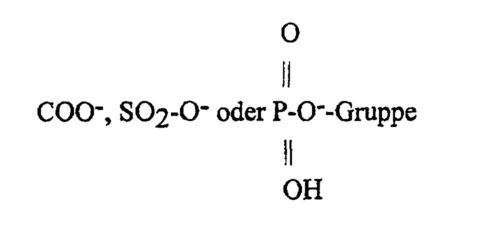 darstellen.

Als besonders geeignet haben sich Homopolymere von 2-Acrylamido-2-methyl-1-propansulfonsäure, 2-Acrylamidoglykolsäure und/oder Copolymere der genannten Monomeren erwiesen.

Die anionische Seitengruppen aufweisenden Polymere haben bevorzugt ein Molekulargewicht von 2 x 10⁴ bis 5 x 10⁶ Dalton, besonders bevorzugt von ungefähr 5 x 10⁵ Dalton auf (Gelpermeationschromatographie):

Diese anionischen Polymere können durch dem Fachmann bekannte Methoden wie Lösungs-, Suspensions- oder Emulsionspolymerisation hergestellt werden (Meth. d. Organ. Chemie (Houben-Weyl), Bd. E 20, Makrom. Stoffe, Thieme Verlag, Stuttgart, 1987). Bevorzugt ist die Lösungsmittelpolymerisation, wie sie beispielsweise in Polymer 31 (1990), 1269-1276 (Huglin und Rego) beschrieben ist. Die Polymerisation kann durch herkömmliche, in wäßrigen Systemen eingesetzte, freie Radikale bildende Initiatoren, insbesondere Peroxide, Persulfate oder Persulfat/Bisulfit oder entsprechende Azoverbindungen beschleunigt werden. Auch dies ist dem Fachmann bekannt.

Die Konzentration eines oder mehrerer der verzweigten Polyanionen liegt zwischen 0.001 bis 5.0 mg/ml, sie beträgt vorzugsweise zwischen 0.005 bis 1.0 mg/ml. Als besonders geeignet hat sich hier ein Bereich von 0.01 bis 0.1 mg/ml erwiesen.

Bei den erfindungsgemäß zuzusetzenden Detergenzien handelt es sich insbesondere um solche mit zwitterionischem und/oder nichtionischem Charakter.

Bevorzugt sind solche Detergenzien, in denen x eine ungerade Zahl zwischen 11 bis 17, y eine Zahl zwischen 1 und 3 und/oder R² einen Sulfonsäure-, Hydroxyethylsulfonsäure-, Hydroxypropylsulfonsäure- oder Carboxylrest oder R¹ Wasserstoff, ein Steroidgerüst, ein Lecithin- oder Lysolecithinrest darstellen.

Insbesondere als geeignet haben sich folgende Detergenzen erwiesen: N-Dodecyl-N,N-dimethylammonio-3-propansulfat bzw. die entsprechende N-Tetradecyloder N-Hexadecyl-Verbindung (Typ "Zwittergent": Zwittergent 3-14, 3-16), N-Dodecyl-N,N-dimethyl-glycin (Empigen BB®), Aminoxide, CHAPS, CHAPSO und α-Lecithin (α-Phosphatidylcholin) bzw. α-Lysolecithin (α-Lysophosphatidylcholin). Sämtliche Detergenzien sind über einschlägige Anbieter erhältlich.

Die zwitterionischen Detergenzien werden allein oder in Kombination mit einem nichtionischen Detergenz wie beispielsweise Pluronic F68 und/oder Tween 20 zugesetzt.

Generell können für das erfindungsgemäße Bestimmungsverfahren Puffersubstanzen verwendet werden, die ihre Pufferkapazität im schwach sauren, neutralen oder leicht alkalischen pH-Bereich haben. Besonders geeignet sind als Puffersubstanzen Natriumacetat und sogenannte Good-Puffer, wie z.B. TRIS·HCl, BIS-TRIS-Methan, MES oder Imidazol. Der pH-Wert für die Bestimmung bei Verwendung dieser Puffersubstanzen ist vorzugsweise zwischen pH 5 und 9 zu wählen, besonders bevorzugt ist ein Bereich von 7.0 bis 8.0 und ganz besonders geeignet ist ein pH-Bereich von 7.3 bis 7.7. Die Konzentration des Puffers sollte zwischen 0.001 bis 0.2 mol/l liegen. Als besonders geeignet hat sich hier ein Konzentrationsbereich von 0,005 bis 0,07 mol/l erwiesen.

Eine bevorzugte Ausführungsform besteht in der Verwendung eines TRIS·HCl-, eines Bis [2- Hydroxyethyl] imino-tris [hydroxymethyl] methan (Bis-TRIS), eines MES- oder eines Imidazol-Puffers in Gegenwart von 2-wertigen Metallionen, wobei die Konzentration des Puffers von 0.001 bis 0.2 mol/l, die Konzentration der Metallsalze von 0.001 - 0.20 mol/l und der pH-Wert 5,5 bis 9,0 betragen. In einer besonders bevorzugten Ausführungsform beträgt die Konzentration des Puffers 0.005 bis 0.07 mol/l, die Konzentration der Metallsalze 0.01 bis 0.03 mol/l und der pH-Wert 7.0 bis 8.0. Generell können die Salze aller bekannten 2-wertigen Metallionen verwendet werden. Bevorzugt verwendet werden Mg^{2+,} Ca^{2+,} Mn ²⁺ und Cu ²⁺. Besonders geeignet haben sich Mg ²⁺ und Ca ²⁺ erwiesen. Die Polyanionkonzentration ist wie oben angegeben zu wählen.

Ein weiterer Gegenstand der Erfindung ist ein Reagenz, welches ein wasserlösliches Polymeres und ein zwitterionisches und/oder ein nichtionisches Detergenz sowie eine im pH-Bereich von 5 bis 9 puffernde Substanz enthält. Die Menge an Polymeren beträgt dabei zwischen ca. 0.005 bis 1.0 mg/ml Reagenzlösung; bevorzugt wird Poly-(2-acrylamido-2-methyl-1-propansulfonsäure) (PAMPS), Polyacrylsäure-(2-phos-pho-1,1-dimethyl-ethylamid) (PAP), Poly-2-acrylamidoglykolsäure (PAAG), Poly-(2-acrylamido-2-methyl-1-propansulfonsäure-CO-2-acrylamido-glykolsäure) [P(AMPS-AAG)] und/oder entsprechende Copolymere und/oder entsprechende Poly(meth) acrylsäureester oder auch Dextransufat, Polyvinylsulfat bzw. Heparin in einem Konzentrationsbereich von 0.01 - 0.1 mg/ml, ganz besonders bevorzugt im Konzentrationsbereich von 0.02 - 0.04 mg/ml zugesetzt.

Die Menge des zuzusetzenden Detergenzes ist sehr vom jeweiligen Detergenztyp abhängig. So wird beispielsweise die Wirksamkeit der VLDL-Entstörung bei Detergenzien mit kürzerer Kettenlänge (x, y < 4) erst bei höheren Konzentrationen erreicht. Als besonders geeignet hat sich der Zusatz eines zwitterionischen Detergenzes in Konzentrationen von ca. 0,002 bis 1,0% (w/v) im Bestimmungsansatz erwiesen.

Als Puffersubstanzen haben sich Natriumacetat, TRIS·HCl, BIS-TRIS, MES und Imidazol als besonders geeignet erwiesen. Die Konzentration des Puffers beträgt vorzugsweise 0.001 - 0.2 mol/l.

Bei den angegebenen Konzentrationen handelt es sich jeweils um Endkonzentrationen, d.h. die Konzentration im jeweiligen Reaktionsansatz.

Der pH-Wert-Bereich des Reagenzes für die Bestimmung von LDL liegt zwischen 5.0 und 9.0, bevorzugt zwischen pH 7.0 und 8.0 oder zwischen pH 7.3 und 7.7. Die Temperatur kann für die Bestimmung zwischen 10 und 40°C variieren; bevorzugt wird bei ca. 37°C gemessen.

Das Volumenverhältnis von zu untersuchender Probe zur Reagenzlösung ist variabel. Verhältnisse von 1:6 bis 1:100 haben sich hier als geeignet erwiesen. Bevorzugt werden ca. 5 Volumenteile einer Serumprobe (z. B. 5 µl) mit 300 Volumenanteilen Reagenzlösung (z. B. 300 µl) miteinander vermischt. Als Probe kommen insbesondere biologische Flüssigkeiten wie Serum. Plasma oder Urin in Betracht.

Des weiteren hat sich als vorteilhaft herausgestellt, daß zunächst zwei getrennte Reagenzlösungen gefertigt werden, wovon die eine ein 2-wertiges Metallion und die zweite ein wasserlösliches polymeres Aggretationsmittel enthält, wobei entweder im ersten Reagenz, das zuerst mit der Probe versetzt wird, oder in beiden Reagenzien ein zwitterionisches und/oder ein nicht-ionisches Detergenz zugesetzt wird.

Die mit einem LDL fällenden Mittel wie beispielsweise Dextransulfat, Polyvinylsulfat oder Heparin bzw. bevorzugt mit einem anionischen Polymeren (PAMPS) und einem oder mehreren der erfindungsgemäßen Detergenzien bewirkte LDL-spezifische Aggregation bzw. Agglutination bietet gegenüber den bekannten Methoden bedeutende Vorteile:
1) Außer LDL gehen andere Apoprotein B-haltige Lipoproteine wie Very Low Density Lipoproteine (VLDL), Lp(a) und/oder Chylomikronen keine Wechselwirkung mit der erfindungsgemäßen Kombination ein und werden somit nicht erfaßt.
2) Hohe Konzentrationen an Triglyceriden haben keinen negativen Einfluß auf das Meßergebnis.
3) Die Bestimmung erfolgt sehr schnell - dazu ohne Probenvorbehandlung - (ca. 1 -10 min);
4) gebildetes Aggregat liegt in einem stabilen Zustand vor und ist daher meßtechnisch direkt und reproduzierbar erfaßbar;
5) die Bestimmung des LDL-Cholesterin verläuft in einem breiten Meßbereich linear (50 - 350 mg/dl LDL-Cholesterin).

Das erfindungsgemäße Verfahren bzw. Reagenz führt somit in bislang nicht bekannter Weise innerhalb von 1 bis 5 Minuten zur vollständigen spezifischen Agglutination der LDL-Partikel, ohne Störung durch erhöhte Triglyceridkonzentrationen und ohne vorherige Abtrennung der anderen Lipoproteinfraktionen, und erlaubt daher die für die Routineanalytik erwünschte Verwendung eines Analyseautomaten bzw. eines einfachen Photometers (turbidimetrisch) zur Bestimmung der LDL-Konzentration bzw. des LDL-Cholesterins. Weiterhin ist es möglich, auch noch das in dem LDL-Agglutinat enthaltende Apoprotein B-100 und/oder andere molekulare Bestandteile der LDL-Partikel zu bestimmen.

Der breite Meßbereich, in dem die erfindungsgemäße Methode ein lineares Meß-. signal aufweist, schließt den diagnostisch relevanten Bereich, insbesondere auch den erhöhter LDL-Werte (> 190 mg/dl), vollständig mit ein, was den weiteren Vorteil mit sich bringt, daß nur eine Einpunkt-Kalibrierung erforderlich ist. Auch lassen sich durch den erweiterten linearen Bereich insbesondere Werte erhöhter LDL-Spiegel präziser quantitativ verfolgen, ohne daß entsprechende aufwendige Bestätigungsverfahren wie Lipoprotein-Elektrophorese bzw. Ultrazentrifugation erforderlich sind.

Das erfindungsgemäße Verfahren bzw. Reagenz agglutiniert neben LDL nicht zusätzlich LDL-ähnliches Lp(a). Dies ist überraschend, da sämtlich bekannte LDL-Fällungsreagenzien auch die Lp(a)-Fraktion zumindest teilweise mitfällen. Somit ist mit der vorliegenden Erfindung darüber hinaus möglich, auch die Lp(a)-Fraktion in einfacher Weise zu bestimmen, indem nach erfindungsgemäßer LDL-Agglutination und -Bestimmung in einer dem Fachmann bekannten Weise der über eine Disulfidbrücke verbundene Lp(a)-Anteil des Lp(a)-Partikels reduktiv abgespalten wird und die so erhaltene LDL-Fraktion, beispielsweise erfindungsgemäß, bestimmt wird. Durch Differenzbildung kann somit der Gehalt an Lipoprotein(a) ermittelt werden.

Es ist ferner anzumerken, daß trotz der bisherigen Lehrmeinung, daß durch Zusatz von Detergenzien die LDL-Fällung stark beeinträchtigt wird, es überraschenderweise möglich ist, durch Zusatz an zwitterionischen Detergenzien, vor allem vom Typ Zwittergent oder durch Gemische von nichtionischen Detergentien, die gleichzeitige Präzipitation von VLDL zu verhindern, ohne daß die spezifische Fällung von LDL beeinträchtigt wird. Besonders wichtig ist es, die optimale Konzentration des Detergenzes im Reaktionsgemisch zu ermitteln. Während bei zu geringen Konzentrationen die Entstörwirkung gleich Null ist, kann bei zu hohen Detergenzkonzentrationen zwar die gleichzeitige Präzipitation von VLDL verhindert, aber auch die vollständige Präzipitation von LDL-Partikeln reduziert werden. Mit dem erfindungsgemäßen Verfahren bzw. Reagenz ist eine störungsfreie LDL-Bestimmung bis ca. 130 mg/dl VLDL-Gehalt, dies entspricht einem Triglyceridgehalt von ca. 800 mg/dl, möglich, während mit den Routinemethoden wie Dextransulfatfällung bereits ab 400 mg/dl Triglyceridgehalt eine deutliche Störung auftritt.

Weiterhin ist auch die Durchführung des erfindungsgemäßen Verfahrens auf Teststreifen oder ähnlichen Matrices möglich, wenn die einzelnen Reaktionskomponenten auf oder in einem Trägermaterial imprägniert oder kovalent gebunden vorliegen. Als Trägermaterial kann ein saugfähiges, quellfähiges oder filmbildendes Trägermaterial, z. B. ein für Teststreifen bekanntes Trägermaterial wie Papier und ähnliche Vliesmaterialien, beispielsweise Teebeutelpapier, verwendet werden. Dabei können die Reaktionskomponenten auf mehrere, in Kontakt stehende Träger verteilt sein oder selbst als Trägermaterial dienen. Kovalent gebundenes Polyanion (z.B. PAMPS) ermöglicht so die vollständige, selektive Abtrennung von LDL auf einem Teststreifen. Durch Messung des Cholesteringehalts der ungebundenen Lipoproteinpartikel und Differenzbildung mit dem Gesamtcholesterinwert kann der LDL-Cholesterinwert ermittelt werden.

### Abkürzungen

- CHAPS =: [3-(3-Cholamidopropyl)-dimethylammonio]-1-propansulfonat
- CHAPSO =: [3-(3-Cholamidopropyl)-dimethylammonio]-2-hydroxy-1-propansulfonat
- MES =: 2-N-Morpholin-ethansulfonsäure

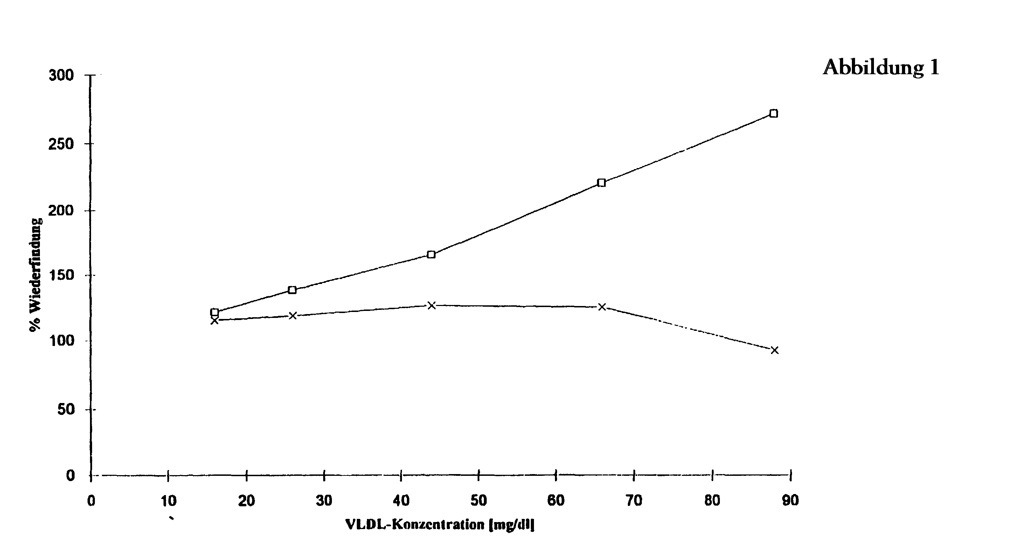

- Abbildung 1:: Wiederfindung LDL (in Prozent) in Abhängigkeit vom VLDL-Gehalt [mg/dl]
-□- ohne Detergenz
-×- 0.005 % Zwittergent 3-14

Die folgenden Beispiele erläutern die Erfindung weiter:

### Beispiel 1

### Einfluß der Zwittergent-Konzentration auf die VLDL-Entstörung im homogenen LDL-Test

- **Reagenzlösung 1:**: 10 mM Bis-Tris-Methan, pH 7,5
12 mM MgCl₂
x % Zwittergent 3 - 14 (= N-Tetradecyl-N,N-dimethylammonio-3-propansulfonat)
- **Reagenzlösung 2:**: 10 mM Bis-Tris-Methan, pH 7, 5
0.05 mg/ml PAMPS

Eine native Probe mit niedrigem VLDL-Gehalt (16 mg/dl) wurde mit einer LDL-freien VLDL-Präparation zunächst verschnitten. 5 µl dieser Probe werden mit 250 µl Reagenzlösung 1 vermischt und 5 Minuten bei 37°C inkubiert. Anschließend werden 50 µl Reagenzlösung 2 zugesetzt und wiederum 5 Minuten bei 37°C inkubiert. Die hierbei entstehende Trübung wird sofort nach der 5-minütigen Inkubationszeit bichromatisch bei 505 nm (Referenzwellenlänge 700 nm) vermessen.

Aus Tabelle 1 geht hervor, daß eine Konzentration von 0,005 und 0,01 % an Zwittergent wirksam ist, d.h. es wurde nahezu eine vollständige Entstörung von VLDL erreicht, während ohne Detergenzzusatz eine deutliche Störung ab 25 mg/dl VLDL auftritt.

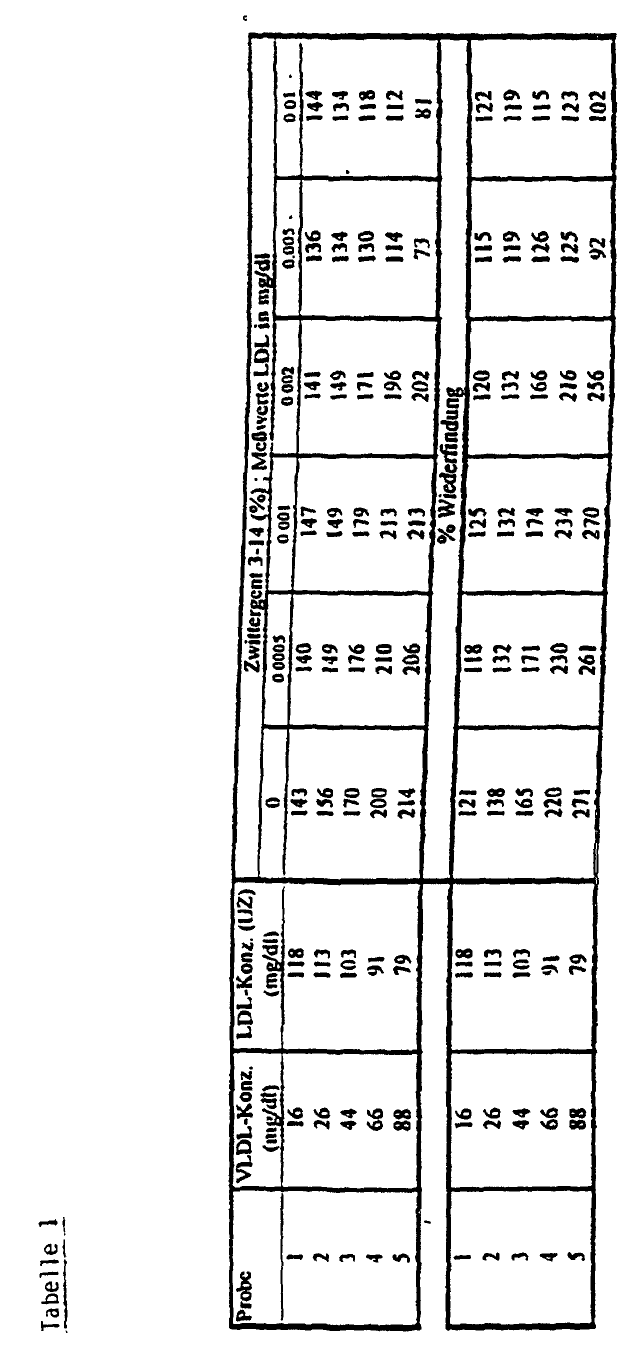

### Beispiel 2

### Wirkung verschiedener zwitterionischer Detergenzien auf die VLDL-Störung im homogenen LDL-Test

- **Reagenzlösung:**: s. Beispiel 1
Folgende Detergenzien wurden neben Zwittergent
3 - 14 verwendet:
Zwittergent 3 - 08
Zwittergent 3 - 10
Zwittergent 3 - 12
Zwittergent 3 - 16

Eine native Probe mit niedrigem VLDL-Gehalt (16 mg/dl) wurde wie in Beispiel 1 beschrieben mit einer LDL-freien VLDL-Präparation verschnitten und vergleichend vermessen.

Tabelle 2 zeigt, daß neben Zwittergent 3 - 14 auch mit Zwittergent 3 - 16 eine sehr gute Entstörung von VLDL-aufgestockter Probe erreicht wird, während die Varianten, welche eine etwas kürzere Alkylgruppe enthalten, in den getesteten Konzentrationen keine Verbesserung bewirkten.

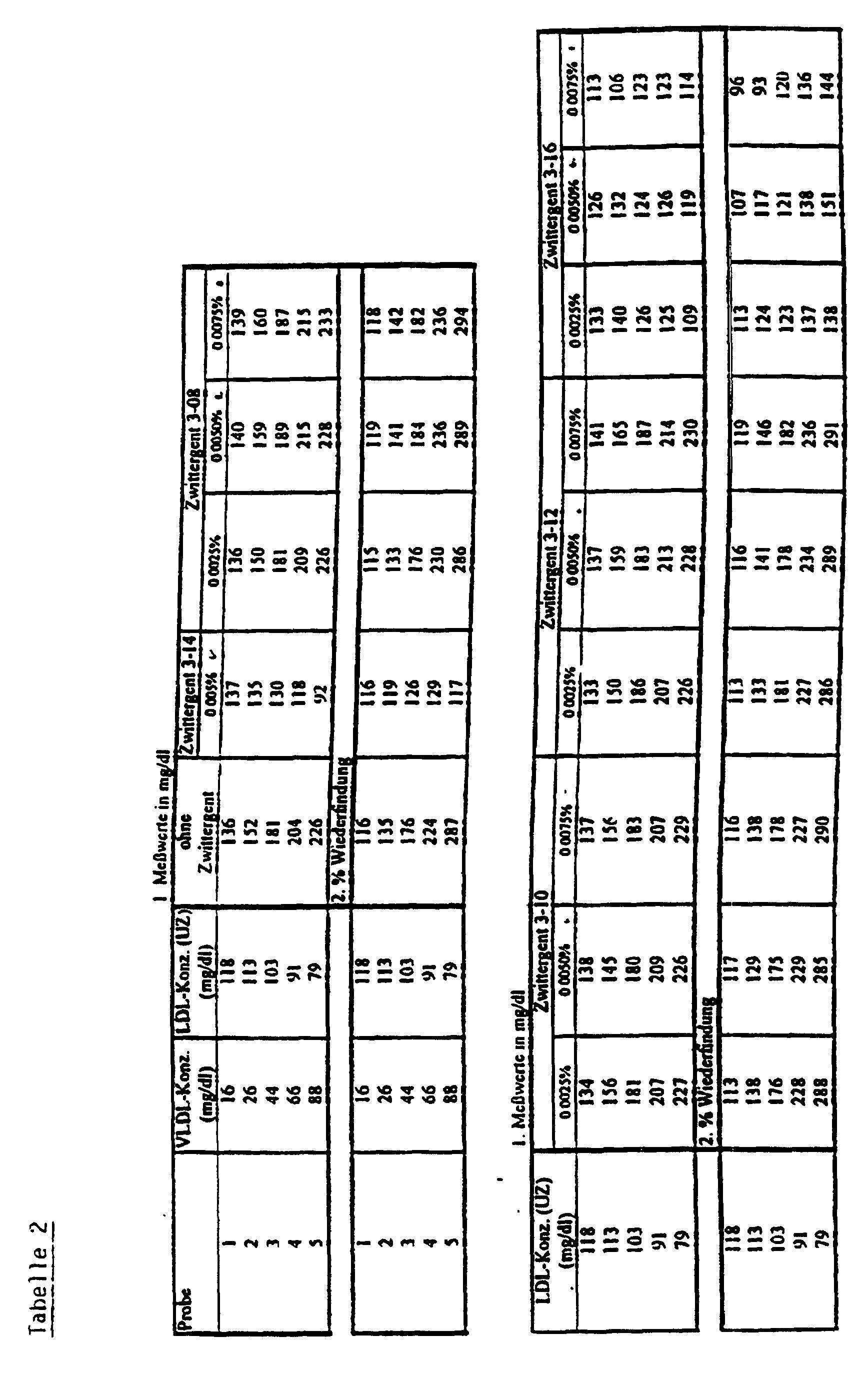

### Beispiel 3

### Wirkung verschiedener zwitterionischer Detergenzien und Detergenzmischungen auf die VLDL-Störung im LDL-Test

Eine VLDL-Präparation ohne LDL-Gehalt mit einem VLDL-Gehalt von 64 mg/dl wurde wie in Beispiel 1 beschrieben vergleichend vermessen. Da kein LDL in der Präparation enthalten ist, muß ein LDL-Wert von 0 (Optimalwert) erzielt werden.
- Reagenz 1:: 10 mM BIS-Tris-Methan pH 7.5
0.03 mg/ml PAMPS
+ Detergenz
- Reagenz 2:: 10 mM Bis-Tris-Methan pH 7.5
120 mM MgCl2
+ Detergenz

**Tabelle 3**

| **Detergenz** | **LDL [mg/dl]** |
|---|---|
| ohne Detergenz | 38.3 |
| 0.005 % Zwittergent 3-14 | 1.0 |
| 0.01 % Chaps | 10.1 |
| 0.01 % Chapso | 10.0 |
| 0.01 % Empigen | 13.8 |
| 0.01 % Phosphatidylcholin | 11.8 |
| 0.01 % Diheptadecanoyl Phosphatidylcholin | 10.5 |
| 0.01 % Dodecyldimethylaminoxid | 8.2 |
| 0.1 % Pluronic F68/0.01 % Tween 20 | -0.6 |

### Beispiel 4

### Einfluß der Konzentration verschiedener Zwittergenttypen auf die VLDL-Entstörung

Es wurde der Einfluß der Detergenzkonzentration der Zwittergenttypen 3-10, 3-12 und 3-14 in nativen Proben mit unterschiedlichen VLDL-Gehalten überprüft. Die LDL- und VLDL-Sollwerte der Proben wurden mit der UZ-Referenzmethode ermittelt.
- Reagenz 1:: 40 mM BIS-Tris-Methan pH 7.5
24 mM MgCl₂
x % Zwittergent
- Reagenz 2:: 40 mM Bis-Tris-Methan pH 7.5
0.15 mg/ml PAMPS
x % Zwittergent

Versuchsdurchführung: siehe Beispiel 1.

Die Ergebnisse in Tabelle 4 belegen, daß Proben bis zu 130 mg/dl VLDL-Gehalt völlig entstört werden können. Bei Verwendung von Zwittergenttypen mit kürzerer Kettenlänge x ist eine höhere Konzentration zur Entstörung notwenig.

**Tabelle 4**

| | Gemessene Konzentration (mg/dl) | | | | | |
|---|---|---|---|---|---|---|
| | Probe 1: | Probe 2 | Probe 3 | Probe 4 | Probe 5 | Probe 6 |
| | LDL: | LDL: | LDL: | LDL: | LDL: | LDL: |
| | 122 mg/dl | 153 mg/dl | 178 mg/dl | 237 mg/dl | 191 mg/dl | 182 mg/dl |
| | VLDL: | VLDL: | VLDL: | VLDL: | VLDL: | VLDL: |
| | 20 mg/dl | 35 mg/dl | 46 mg/dl | 55 mg/dl | 65 mg/dl | 134 mg/dl |
| Zwittergent 3-10 | | | | | | |
| 0.001 % | 130 | 181 | 199 | 260 | 232 | 314 |
| 0.005 % | 133 | 180 | 198 | 263 | 230 | 313 |
| 0.025% | 135 | 172 | 198 | 263 | 240 | 313 |
| 0.05 % | 136 | 182 | 207 | 258 | 249 | 318 |
| 0.25 % | 118 | 145 | 185 | 238 | 193 | 213 |
| | | | | | | |

| Zwittergent 3-12 | | | | | | |
|---|---|---|---|---|---|---|
| 0.001 % | 133 | 190 | 205 | 252 | 248 | 329 |
| 0.005 % | 134 | 186 | 206 | 253 | 238 | 305 |
| 0.025 % | 134 | 177 | 188 | 237 | 195 | 250 |
| 0.05 % | 122 | 154 | 179 | 235 | 189 | 190 |
| | | | | | | |

| Zwittergent 3-14 | | | | | | |
|---|---|---|---|---|---|---|
| 0.001 % | 142 | 171 | 207 | 264 | 245 | 310 |
| 0.005 % | 129 | 160 | 179 | 237 | 189 | 186 |

| | | | % Wiederfindung | | | |
|---|---|---|---|---|---|---|
| | Probe 1: | Probe 2 | Probe 3 | Probe 4 | Probe 5 | Probe 6 |
| | LDL: | LDL: | LDL: | LDL: | LDL: | |
| | 122 mg/dl | 153 mg/dl | 178 mg/dl | 237 mg/dl | 191 mg/dl | 182 mg/dl |
| | VLDL: | VLDL: | VLDL: | VLDL: | VLDL: | VLDL: |
| | 20 mg/dl | 35 mg/dl | 46 mg/dl | 55 mg/dl | 65 mg/dl | 134 mg/dl |
| Zwittergent 3-10 | | | | | | |
| 0.001 % | 107 | 118 | 112 | 109 | 121 | 173 |
| 0.005 % | 109 | 118 | 111 | 111 | 120 | 172 |
| 0.025% | 111 | 112 | 111 | 111 | 126 | 172 |
| 0.05 % | 111 | 119 | 116 | 109 | 130 | 175 |
| 0.25 % | 97 | 98 | 104 | 100 | 101 | 117 |
| | | | | | | |

| Zwittergent 3-12 | | | | | | |
|---|---|---|---|---|---|---|
| 0.001 % | 109 | 124 | 115 | 106 | 130 | 181 |
| 0.005 % | 110 | 121 | 116 | 107 | 125 | 161 |
| 0.025 % | 110 | 116 | 106 | 100 | 102 | 137 |
| 0.05 % | 100 | 101 | 101 | 99 | 99 | 104 |
| | | | | | | |

| Zwittergent 3-14 | | | | | | |
|---|---|---|---|---|---|---|
| 0.001 % | 116 | 112 | 116 | 111 | 128 | 170 |
| 0.005 % | 106 | 105 | 101 | 100 | 99 | 102 |

## Patentansprüche

1. Verfahren zur spezifischen Bestimmung der LDL-Fraktion in Gegenwart anderer Lipoproteine des Serums durch Zusatz eines Puffers, eines LDL fällenden oder eines wasserlöslichen polymeren LDL-aggregierenden Mittels sowie eines zwitterionischen und/oder eines nichtionischen Detergenzes der allgemeinen Formel (II) 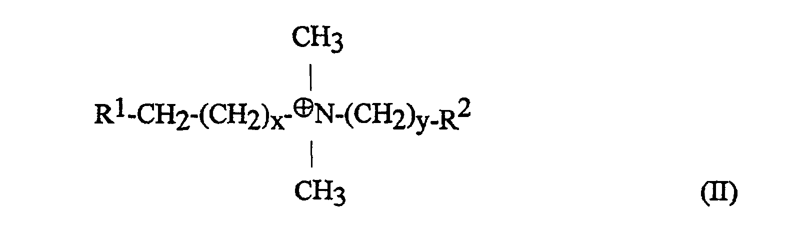 wobei x eine Zahl von 1 bis 20, y eine Zahl von 0 bis 8,
R¹ Wasserstoff, eine Cholamido- oder eine über einen Phosphatrest gebundene Mono- oder Di-Glyceridgruppe ist,
R² Wasserstoff, eine Hydroxygruppe bzw. ein Oxyanion, ein Säurerest, eine Hydroxyalkylsulfonsäure- oder eine Carbonylgruppe ist,
und direkte Meßung des LDL-Aggregats.

2. Verfahren nach Anspruch 1, **dadurch gekennzeichnet, daß** als LDL-aggregierendes Mittel Dextransulfat, Polyvinylsulfat, Heparin oder ein wasserlösliches Polymeres mit Alkansulfonsäure-, Alkanphosphonsäure- und/oder Alkancarbonsäure-Seitengruppen und einem Molekulargewicht von 20.000 bis 5.000.000 Dalton (Gelpermeationschromatographie) und als Puffer Natriumacetat, TRIS·HCl, BIS-Tris-Methan oder MES und/oder Imidazol zugesetzt wird und der pH-Wert zwischen 5 und 9 liegt.

3. Verfahren nach Anspruch 1 oder 2, **dadurch gekennzeichnet, daß** ein Detergenz der allgemeinen Formel (II), in der x eine ungerade Zahl zwischen 11 und 17, y eine Zahl zwischen 1 und 3 und/oder R² einen Sulfonsäure-, Carboxyl-, Hydroxyethylsulfonsäure- oder Hydroxypropylsulfonsäurerest und R¹ Wasserstoff darstellen, zugesetzt wird.

4. Verfahren nach einem der Ansprüche 1 bis 3, **dadurch gekennzeichnet, daß** man das Detergenz N-Decyl, N-Dodecyl, N-Tetradecyl- oder N-Hexadecyl-N,N-dimethylammonio-3-propansulfonat oder ein entsprechendes Derivat zusetzt.

5. Verfahren nach Anspruch 1-4, **dadurch gekennzeichnet, daß** als Detergenz Pluronic F68 und/oder Tween 20 zugesetzt wird.

6. Verfahren nach einem der Ansprüche 1 bis 5, **dadurch gekennzeichnet, daß** das LDL-aggregierende Mittel in einer Konzentration von 0.005 bis 1.0 mg/ml in der Reaktionsmischung oder/und das Detergenz in einer Konzentration von 0,001 bis 0,1 % (w/v).

7. Verfahren nach einem der Ansprüche 1 bis 6, **dadurch gekennzeichnet, daß** Salze 2-wertiger Metallionen in einer Konzentration von 0.001 bis 0.20 mol/l zugesetzt werden.

8. Verfahren nach einem der Ansprüche 1 bis 8, **dadurch gekennzeichnet, daß** bei einer Temperatur von 10 bis 40°C gearbeitet wird und das LDL-Aggregat turbidimetrisch bestimmt wird.

9. Verfahren nach einem der Ansprüche 1 bis 8, **dadurch gekennzeichnet, daß** Low Density-Lipoproteine, LDL-Cholesterin, LDL-Apolipoprotein B oder sonstige molekularen Bestandteile der LDL-Partikel bestimmt werden.

10. Reagenz zur spezifischen Bestimmung von LDL in Gegenwart anderer Lipoproteine des Serums enthaltend (1) Dextransulfat, Polyvinylsulfat ein Alkansulfonsäure-, Alkanphosphonsäure- und/oder Alkancarbonsäure-Seitengruppen aufweisendes Polymer, (2) einen Puffer für pH 5 bis 9 und (3) ein Detergenz, **dadurch gekennzeichnet, daß** das Polyanion ein Molekulargewicht von 20.000 biss 5.000.000 Dalton (Gelpermeationschromatographie) aufweist, das Detergenz eine Verbindung entsprechend der allgemeinen Formel (II) 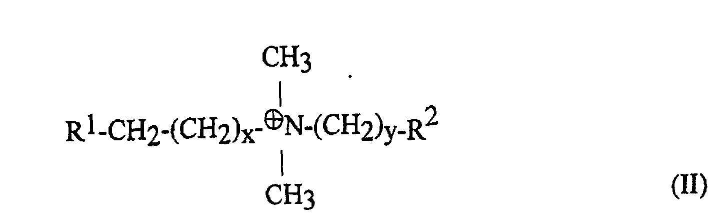 wobei x eine Zahl von 1 bis 20, y eine Zahl von 0 bis 8,
R¹ Wasserstoff, eine Cholamido- oder eine über einen Phosphatrest gebundene Mono- oder Di-Glyceridgruppe ist,
R² Wasserstoff, eine Hydroxygruppe bzw. ein Oxyanion, ein Säurerest, eine Hydroxyalkylsulfonsäure- oder eine Carboxylgruppe ist,
und der Puffer Natriumacetat, TRIS·HCl, Bis-TRIS-Methan oder MES und/oder Imidazol ist.

11. Reagenz nach Anspruch 10, **dadurch gekennzeichnet, daß** das Detergenz N-Decyl-, N-Dodecyl-, N-Tetradecyl- oder N-Hexadecyl-N,N-dimethylammonio-3-propansulfonat oder ein Derivat davon ist.

12. Reagenz nach den Ansprüchen 10 oder 11, **dadurch gekennzeichnet, daß** als Detergenz Pluronic F68 und/oder Tween 20 enthalten ist.

13. Reagenz nach einem der Ansprüche 10 oder 11, **dadurch gekennzeichnet, daß** es auf oder in einem blattförmigen Trägermaterial imprägniert vorliegt.

14. Verwendung einer Verbindung entsprechend der allgemeinen Formel (II) 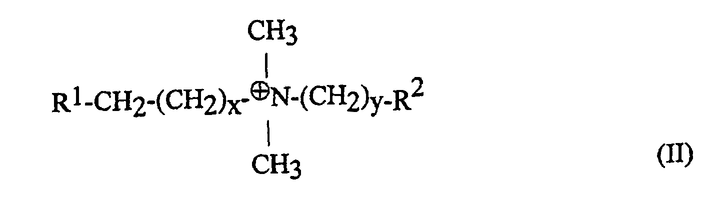 wobei x eine Zahl von 1 bis 20, y eine Zahl von 0 bis 8,
R¹ Wasserstoff, eine Cholamido- oder eine über einen Phosphatrest gebundene Mono- oder Di-Glyceridgruppe ist,
R² Wasserstoff, eine Hydroxygruppe bzw. ein Oxyanion, ein Säurerest, eine Hydroxyalkylsulfonsäure- oder eine Carboxylgruppe ist, zur selektiven Bestimmung von LDL in einer biologischen Probe.

## Claims

1. Method for the specific determination of the LDL fraction in the presence of other serum lipoproteins by adding a buffer, an LDL-precipitating or a watersoluble polymeric LDL-aggregating agent and a zwitterionic and/or a non-ionic detergent of the general formula (II) 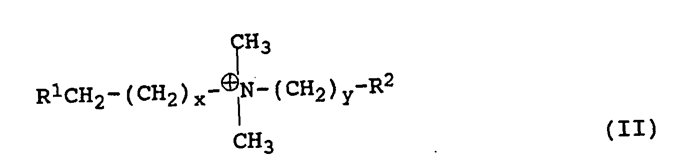 in which x is a number from 1 to 20, y is a number from 0 to 8,
R¹ is hydrogen, a cholamido group or a mono- or diglyceride group bound via a phosphate residue,
R² is hydrogen, a hydroxy group or an oxyanion, an acid residue, a hydroxyalkylsulfonic acid group or a carbonyl group,
and direct measurement of the LDL aggregate.

2. Method as claimed in claim 1, wherein dextran sulfate, polyvinyl sulfate, heparin or a watersoluble polymer containing alkanesulfonic acid side groups, alkanephosphonic acid side groups and/or alkanecarboxylic acid side groups having a molecular weight of 20,000 to 5,000,000 Dalton (gel permeation chromatography) is added as an LDL-aggregating agent and sodium acetate, Tris-HCl, Bis-Tris-methane or MES and/or imidazole is added as the buffer and the pH value is between 5 and 9.

3. Method as claimed in claim 1 or 2, wherein a detergent of the general formula (II) is added in which x is an odd number between 11 and 17, y is a number between 1 and 3 and/or R² a sulfonic acid group, a carboxyl group, a hydroxyethylsulfonic acid group or a hydroxypropylsulfonic acid residue and R¹ is hydrogen.

4. Method as claimed in one of the claims 1 to 3, wherein the detergent N-decyl, N-dodecyl, N-tetradecyl or a N-hexadecyl-N,N-dimethylammonio-3-propane sulfonate or a corresponding derivative is added.

5. Method as claimed in claim 1 to 4, wherein Pluronic F68 and/or Tween 20 are added as the detergent.

6. Method as claimed in one of the claims 1 to 5, wherein the LDL-aggregating agent is present at a concentration of 0.005 to 1.0 mg/ml in the reaction mixture and/or the detergent is present at a concentration of 0.001 to 0.1 % (w/v).

7. Method as claimed in one of the claims 1 to 6, wherein salts of divalent metal ions are added at a concentration of 0.001 to 0.20 mol/l.

8. Method as claimed in one of the claims 1 to 8, wherein the working temperature is between 10 and 40°C and the LDL aggregate is determined turbidimetrically.

9. Method as claimed in one of the claims 1 to 8, wherein low density lipoproteins, LDL cholesterol, LDL apolipoprotein B or other molecular components of the LDL particles are determined.

10. Reagent for the specific determination of LDL in the presence of other serum lipoproteins containing (1) dextran sulfate, polyvinyl sulfate, a polymer having alkanesulfonic acid, alkanephosphonic acid and/or alkanecarboxylic acid side groups, (2) a buffer for pH 5 to 9, and (3) a detergent, wherein the polyanion has a molecular weight of 20,000 to 5,000,000 Dalton (gel permeation chromatography), the detergent is a compound of the general formula (II) 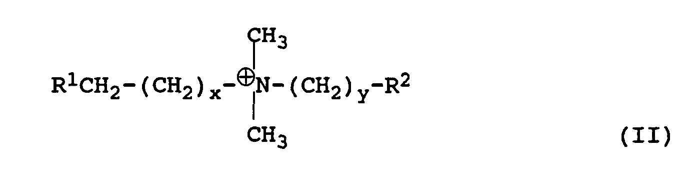 in which x is a number from 1 to 20, y a number from 0 to 8,
R¹ is hydrogen, a cholamido group or a mono- or diglyceride group bound via a phosphate residue,
R² is hydrogen, a hydroxy group or an oxyanion, an acid residue, a hydroxyalkylsulfonic acid group or a carboxyl group,
and the buffer is sodium acetate, Tris-HCl, Bis-Tris-methane or MES and/or imidazole.

11. Reagent as claimed in claim 10, wherein the detergent is N-decyl, N-dodecyl, N-tetradecyl- or a N-hexadecyl-N,N-dimethylammonio-3-propanesulfonate or a derivative thereof.

12. Reagent as claimed in one of the claims 10 or 11, wherein it contains Pluronic F68 and/or Tween 20 as the detergent.

13. Reagent as claimed in one of the claims 10 or 11, wherein it is present on or in a leaf-like carrier material in an impregnated form.

14. Use of a compound corresponding to the general formula (II) 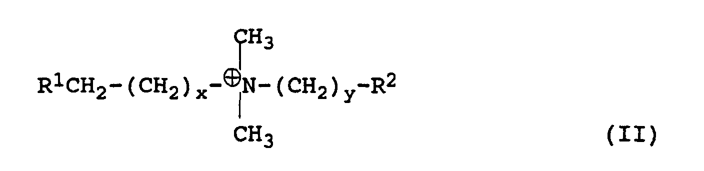 in which x is a number from 1 to 20, y is a number from 0 to 8,
R¹ is hydrogen, a cholamido group or a mono- or diglyceride group bound via a phosphate residue,
R² is hydrogen, a hydroxy group or an oxyanion, an acid residue, a hydroxyalkylsulfonic acid group or a carboxyl group for the selective determination of LDL in a biological sample.

## Revendications

1. Procédé pour la détermination spécifique de la teneur en LDL en présence d'autres lipoprotéines du sérum, par addition d'un tampon, d'un agent précipitant le LDL ou d'un agent polymère soluble dans l'eau agglomérant le LDL, ainsi que d'un détergent zwitterionique et/ou d'un détergent non-ionique de formule générale (II) 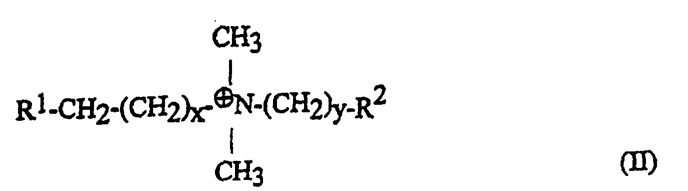 dans laquelle
x représente un nombre de 1 à 20 et y un nombre de 0 à 8,
R¹ représente l'hydrogène, un groupe cholamido glycéride ou un groupe mono ou diglycéride lié par un résidu phosphate,
R² représente l'hydrogène, un groupe hydroxyle ou un oxyanion, un résidu acide, un groupe acide hydroxyalkylsulfonique ou un groupe carbonyle,
et par mesure directe de l'agglomérat de LDL.

2. Procédé selon la revendication 1, **caractérisé en ce que** comme agent d'agglomération du LDL, on utilise du sulfate de dextrane, du poly(sulfate de vinyle), de l'héparine ou un polymère soluble dans l'eau présentant des groupes acide alcanesulfonique, acide alcanephosphonique et/ou acide alcanecarboxylique, et d'un poids moléculaire de 20 000 à 5 000 000 Dalton (par chromatographie par perméation d'un gel), et comme tampon on ajoute de l'acétate de sodium, du TRIS-HCl, du BIS-tris-méthane ou du MES et/ou de l'imidazole, et la valeur du pH est comprise entre 5 et 9.

3. Procédé selon la revendication 1 ou 2, **caractérisé en ce que** l'on ajoute un détergent de formule générale (II) dans laquelle x représente un nombre impair compris entre 11 et 17, y un nombre compris entre 1 et 3, et/ou R² représente un résidu acide sulfonique, acide carboxylique, acide hydroxyéthylsulfonique ou acide hydroxypropylsulfonique, et R¹ représente l'hydrogène.

4. Procédé selon l'une des revendications 1 à 3, **caractérisé en ce que** l'on ajoute le détergent N,N-diméthylammonio-3-propanesulfonate de N-décyle, de N-dodécyle, de N-tétradécyle ou de N-hexadécyle ou un dérivé correspondant.

5. Procédé selon les revendications 1 à 4, **caractérisé en ce que** comme détergent on ajoute du Pluronic F68 et/ou du Tween 20.

6. Procédé selon l'une des revendications 1 à 5, **caractérisé en ce que** l'agent d'agglomération du LDL présente une concentration de 0,005 à 1,0 mg/ml dans le mélange de réaction, et/ou le détergent présente une concentration de 0,001 à 0,1% (poids/volume).

7. Procédé selon l'une des revendications 1 à 6, **caractérisé en ce que** l'on ajoute des sels d'ions métalliques bivalents à une concentration de 0,001 à 0,20 mole/litre.

8. Procédé selon l'une des revendications 1 à 8, **caractérisé en ce que** l'on travaille à une température de 10 à 40° C et **en ce que** l'agglomérat de LDL est déterminé par turbidimétrie.

9. Procédé selon l'une des revendications 1 à 8, **caractérisé en ce que** des lipoprotéines à basse densité, des LDL-cholestérol, des LDL-apolipoprotéines B ou d'autres composants moléculaires des particules de LDL sont déterminés.

10. Réactif en vue de la détermination spécifique de LDL en présence d'autres lipoprotéines du sérum, contenant (1) du sulfate de dextrane, du poly(sulfate de vinyle), un polymère présentant des groupes latéraux acide alcanesulfonique, acide alcanephosphonique et/ou acide alcanecarboxylique, (2) un tampon à pH 5 à 9 et (3) un détergent, **caractérisé en ce que** le polyanion présente un poids moléculaire de 20 000 à 5 000 000 Dalton (chromatographie par perméation d'un gel), le détergent étant un composé correspondant à la formule générale (II) 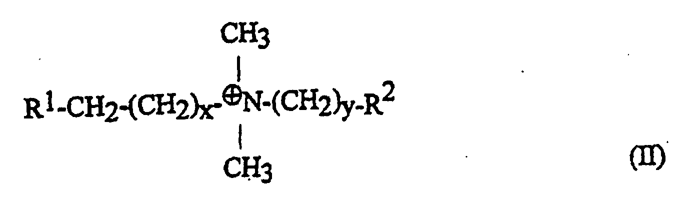 dans laquelle
x représente un nombre de 1 à 20 et y un nombre de 0 à 8,
R¹ représente l'hydrogène, un groupe cholamido glycéride ou un groupe mono ou diglycéride lié par un résidu phosphate,
R² représente l'hydrogène, un groupe hydroxyle ou un oxyanion, un résidu acide, un groupe acide hydroxyalkylsulfonique ou un groupe carboxyle,
le tampon étant l'acétate de sodium, le TRIS-HCI, le bis-tris-méthane, le MES et/ou l'imidazole.

11. Réactif selon la revendication 10, **caractérisé en ce que** le détergent est le N,N-diméthylammonio-3-propanesulfonate de N-décyle, de N-dodécyle, de N-tétradécyle ou de N-hexadécyle ou un dérivé de ceux-ci.

12. Réactif selon la revendication 10 ou 11, **caractérisé en ce qu'**il contient comme détergent du Pluronic F68 et/ou du Tween 20.

13. Réactif selon l'une des revendications 10 ou 11, **caractérisé en ce qu'**il se présente sur un matériau de support en feuille ou imprègne un matériau de support en feuille.

14. Utilisation d'un composé correspondant à la formule générale (II) 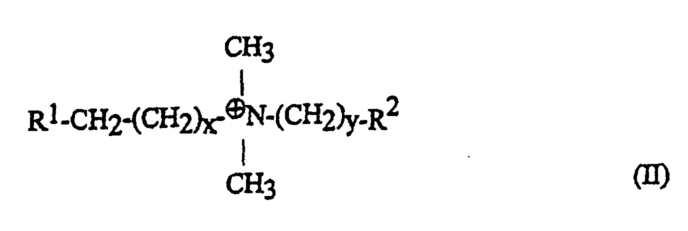 dans laquelle
x représente un nombre de 1 à 20 et y un nombre de 0 à 8,
R¹ représente l'hydrogène, un groupe cholamido glycéride ou un groupe mono ou diglycéride lié par un résidu phosphate,
R² représente l'hydrogène, un groupe hydroxyle ou un oxyanion, un résidu acide, un groupe acide hydroxyalkylsulfonique ou un groupe carboxyle, pour la détermination sélective de LDL dans un échantillon biologique.
